# EUROPEAN PATENT APPLICATION

(11) **EP 4 506 949 A1**
(43) Date of publication of application: **12.02.2025**
(21) Application number: 23720924.2
(22) Date of filing: 28.03.2023
(51) Int. Cl.: G16C 20/50, G16B 15/30

(54) **METHOD AND SYSTEM FOR THE CHARACTERIZATION OF DYNAMIC INTERACTIONS BETWEEN DRUGS AND IKR OR HERG CHANNELS**

(30) Priority: 01.04.2022 ES 202230300
(71) Applicant: Universitat Politècnica de València, 46022 Valencia (ES)
(72) Inventor: ROMERO PÉREZ, Lucía, 46022 Valencia (Valencia) (ES); ESCOBAR ROPERO, Fernando, 46022 Valencia (Valencia) (ES); GOMIS-TENA DOLZ, Julio, 46022 Valencia (Valencia) (ES); SAIZ RODRÍGUEZ, Francisco Javier, 46022 Valencia (Valencia) (ES)
(74) Representative: TRBL Intellectual Property
(86) International application number: PCT/ES2023/070195
(87) International publication number: WO 2023/187237

(57) **Abstract**

The invention relates to a method for the characterization of dynamic interactions between drugs and I_{Kr} or hERG channels, comprising the steps of: modelling the interaction of drugs with I_{Kr} or hERG; selecting the drugs by means of a simulation of compounds distributed in groups with different kinetics and affinities for the conformational states of the I_{Kr} or hERG channel; and defining a plurality of voltage clamp protocols, obtaining their concentration-response distributions. The method further comprises obtaining values of drug concentration at which the half blocking of a corresponding ionic current (IC₅₀) takes place and of the corresponding time constants (τ) for each voltage clamp protocol and for the simulated compounds; and characterizing the compounds according to the values obtained.

## Description

### FIELD OF THE INVENTION

The present invention is comprised in the field of drug development technologies and computational sciences. More specifically, the invention relates to a method for modelling and characterizing dynamic interactions of compounds with I_{Kr} channels (delayed rectifier potassium current) or hERG channels (human ether-a-go-go-related gene).

### BACKGROUND OF THE INVENTION

The discovery and development of new drugs has traditionally been based on trial-and-error procedures. However, in practice these procedures are often inefficient, excessively lengthy or risky, both from a health point of view and from the point of view of investment in private or public research. This is due to the fact that, in the case of many medicines developed, they do not reach the market because of their harmful side effects on health and, specifically for the heart, due to potential adverse effects such as ventricular arrhythmias. Until now, preclinical evaluation of the cardiac safety of new drugs has been based, mainly, on the study of in vitro blockade in the hERG gene (encoding the pore-forming α-subunit of the fast component of the I_{Kr} current channels in humans) produced by the compound under study, and in vivo prolongation of the QT interval (measurement of time between the beginning of the Q wave and the end of the T wave in an electrocardiogram). Both phenomena have been related to the onset of polymorphic ventricular tachycardia ("Torsades de Pointes" or TdP).

Although these safety tests have been successful in preventing potentially harmful drugs from reaching the market, they have also prevented the development of potentially useful compounds. In fact, there are drugs such as verapamil, recognized as a potent I_{Kr} current blocker, which is not associated with the development of TdP.

Recent studies have proven that the evaluation of the safety of new drugs improves when their dynamics and kinetics are taken into account. For example, the dependence of the interaction between a compound and a specific channel with the conformational state in which it is found can significantly alter the values of the drug concentration at which the half blocking of an ionic current (said concentration referred to as IC₅₀) takes place. In turn, said values can also vary depending on the stimulation and voltage-clamp protocol used to measure them.

Moreover, the *"Comprehensive In vitro Proarrhythmia* Assay" (CiPA) initiative, led by the US Food and Drug Administration (FDA), has emerged in recent years, and it seeks to improve the techniques for the discovery and development of drugs by means of defining new paradigms, based on computational models proposed as the main tools for predicting the risk of arrhythmias induced by new drugs. This initiative also places an emphasis on a more detailed pharmacodynamic component. Conventionally, the formulation of the actions of drugs in ion channels is simplified by means of a simple pore blocking model, referred to as a static model, using IC₅₀ coefficients and Hill coefficients to reduce the conductance of each ionic current. These static models are simple but not excessively realistic because, as mentioned above, the compound blocking potency depends on the experimental conditions used to elucidate them. In this scope, dynamic models modelling the kinetics of the drug binding and unbinding to the channel in the different states, such as Markov models, are considered more realistic and provide intrinsic information, which does not depend on the experimental protocol. In this sense, the articles published by Z. Li et al. "Improving the in silico assessment of proarrhythmia risk by combining hERG (human ether- a-go- go- related gene) channel- drug binding kinetics and multichannel pharmacology", Circ. Arrhythm. Electrophysiol. 10, e004628 (2017) and Z. Li et al. "Quantitative Systems Pharmacology Models for a New International Cardiac Safety Regulatory Paradigm: An Overview of the Comprehensive In Vitro Proarrhythmia Assay In Silico Modelling Approach", CPT Pharmacometrics, Syst, Pharmacol (2019), 8, 371-379, are relevant.

Therefore, the current technique proposed by CiPA for obtaining dynamic models of I_{Kr}- or hERG-blocking drugs uses a single Markov chain, from which certain parameters (such as Kₘₐₓ, Kᵤ, EC50ₙ, N, V_{half-trap}) must be adjusted, which makes it possible to distinguish drugs with different binding kinetics captured by a dynamic voltage clamp protocol. However, this technique is showing many problems. For example, many of the proposed drug models do not achieve full blocking of the current, even at high concentrations. Likewise, algorithms for setting parameters are generally excessively complex, and furthermore, the dependence with the seed used to obtain them can condition their results, which limits their use to people already familiar with methods for setting parameters. Finally, the CiPA voltage clamp protocols involve rather long times as compared with typical stability times of cells under those circumstances.

As a consequence of these limitations, in the present technical field it is necessary to develop new modelling methods and systems as alternatives to those that are known which make it possible, among other characteristics and advantages:
- To automatically obtain dynamic models of the interaction, without the need for the intervention of expert modelling or parameter setting personnel.
- To generate the models in short times and without the need for a high computational resource demand.
- To provide a wide variety of generated models.
- To consider that the binding and unbinding of the drug to the channel can take place in any state of the channel.
- To implement voltage clamp protocols that are more suitable than those currently used to produce models of the drugs.

The present invention intends to meet the mentioned need by means of a novel method and system for the automatic modelling and characterization of dynamic interactions between drugs and I_{Kr} or hERG channels.

### BRIEF DESCRIPTION OF THE INVENTION

As described in the background section, to overcome the limitations of the prior art described above, the object of the present invention is to provide an improved technology for automatically characterizing and modelling dynamic interactions between new drugs and I_{Kr} or hERG channels. The present invention can also be used for modelling the dynamic interactions between any agent and I_{Kr} or hERG channels. The term agent includes drugs, antibodies, small molecules, pharmaceutical compounds, peptides, or proteins, among others. Its use is not restricted either to the study of cardiac safety, but rather can be used in other applications, such as the study of the efficacy of antiarrhythmic drugs, for example.

More specifically, a first object of the invention relates to a computer-implementable method for the characterization of dynamic interactions of drugs with I_{Kr} or hERG channels, wherein said method comprises performing the next steps:
- modelling the interaction between I_{Kr} or hERG and the drugs, wherein said modelling comprises generating a Markov chain model;
- selecting a set of drugs to be characterized by means of a simulation of a plurality of compounds distributed in groups with different kinetics and affinities for the conformational states of the I_{Kr} or hERG channel, wherein the distribution of the groups is based on the following criteria:
   ∘ the states to which the drug binds and unbinds and the preferred binding state;
   ∘ the ratio between the dissociation rate for the preferred and for the non-preferred state, which is arbitrarily set in a plurality of threshold values;
   o the ability of bound channels to change their conformational state without becoming separated from the compound;
- defining a plurality of electrical cell stimulation voltage clamp protocols, with their corresponding concentration-response distributions being obtained.

Advantageously, the method of the invention further comprises the next steps:
- obtaining values of drug concentration at which the half blocking of an ionic current (IC₅₀) takes place and of the time constants (τ) of the normalized tail current (that is, of normalized tail current amplitudes for each sweep of the voltage clamp protocol plotted as function of time) upon application of a value of drug concentration substantially equal to the corresponding IC₅₀ for each voltage clamp protocol and for the plurality of compounds simulated in the step of modelling drugs;
- interpolating the IC₅₀ values with a line based on each of the threshold values, on whether or not the channels bound to the compound can change their state while the compound is bound, the states to which the drug binds and unbinds, and on its preferred binding state;
- interpolating the τ values with a line based on each of the threshold values, on whether or not the channels bound to the compound can change state while the compound is bound, the states to which the drug binds and unbinds, and on its preferred binding state;
- classifying the compounds by performing the following sub-steps, performed by means of SVMs and linear interpolation:
   ∘ obtaining the difference in dissociation rates between the preferred state and the other states where each compound is bound, and classifying the compounds according to their dissociation rate with respect to the non-preferred binding state, wherein each compound is assigned to the group corresponding to the closest interpolated line;
   ∘ assigning each compound to the group corresponding to the interpolated line of its closest time constant (τ), calculating the distance between each compound and all the lines, and determining if the drug allows the drug bound channels to change their state if the closest line corresponds with compounds that have this characteristic; and
   ∘ assigning each compound to a class, characterizing it based on the states where it binds and unbinds, and on its preferred binding state, according to the results obtained in the previous sub-steps.

In a preferred embodiment of the invention, during the step of selecting drugs, the group distribution criterion relating to the ability of drug bound channels to change their conformational state without becoming separated from the compound comprises the formulation of a Markov chain model referred to as stuck if said ability is exhibited, or of a Markov chain model referred to as unstuck if said ability is not exhibited.

In a preferred embodiment of the invention, the step of modelling the interaction between the compound and I_{Kr} or hERG comprises the implementation of a Markov chain model with five drug-free states: three closed states (C₃, C₂, C₁), an open state (O), and an inactivated state (I), and up to five additional drug-containing states (C_{3d}, C_{2d}, C_{1d}, O_{d}, and I_{d}).

More preferably, the distribution of groups during the step of modelling drugs comprises the following criteria:
- the states to which the drug binds and unbinds and the preferred binding state;
- the ratio between the dissociation rate of the preferred and of the non-preferred state, which is arbitrarily set in a plurality of threshold values;
- the ability of drug bound channels to change their conformational state without becoming separated from the compound, distinguishing between an unstuck model and a stuck model.

Even more preferably in the method of the invention, the distinction of the compounds according to their binding state comprises the following classes: Open, Inactivated, Closed, OpenI, InactivatedO, OI, OpenC, ClosedO, CO, OpenCl, InactivatedCO, ClosedOl, and COI, wherein said classes correspond to the following binding states: Open, Inactivated, and Closed are compounds interacting only in the open, inactivated, and closed states, respectively. OpenI, InactivatedO, and OI are drugs binding to the open and inactivated states, with preference for the open, inactivated state, and with the same preference for both states, respectively. OpenC, ClosedO, and CO represent drugs binding to the closed and open states simultaneously, but with a higher affinity for the open, closed state and with an identical affinity for both states, respectively. Lastly, OpenCl, InactivatedCO, ClosedOl, and COI are compounds binding simultaneously to the three states with a higher affinity for the open, inactivated, or closed states and with the same affinity for all of them, respectively.

In another preferred embodiment of the invention, the step of modelling the interaction with I_{Kr} or hERG comprises creating additional check groups using a random seed and generating variants in which the channels bound to the compound may or may not change state without becoming separated.

In another preferred embodiment of the invention, the step of defining electrical stimulation protocols comprises the use of protocols comprising, for a given value of temperature and a given value of intracellular and extracellular potassium concentration:
- a variable voltage conditioning pulse;
- a test pulse;
- optionally, a pre-pulse.

More preferably, the electrical stimulation protocols comprise protocols P40, P0, and P-80.

Even more preferably, the step of defining electrical stimulation protocols comprises obtaining concentration-response distributions by means of plotting the normalized peak tail current in the steady state as a function of the common logarithm of the concentration of the drug, and the evolution of the normalized peak tail current of I_{Kr} or hERG at concentration IC₅₀, is analyzed based on the number of pulses or on time.

In another preferred embodiment of the invention, the method comprises an additional step of optimization where, once the compounds are characterized, the association and dissociation constants for each state in which each compound interacts with the channel are calculated and processed by means of an iterative optimization algorithm. More preferably in the method of the invention, the iterative optimization algorithm is based on the Nelder-Mead simplex algorithm.

A second object of the invention relates to a computing system comprising software and/or hardware means adapted for implementing a method according to any of the embodiments described herein.

A third object of the invention relates to a computer program comprising instructions configured for being executed in a method according to any of the embodiments described herein, in a corresponding computing system.

In the scope of interpretation of the invention, the expression "substantially", in reference to any term, is to be understood as identical to said term or comprised within a ±25% margin of variation.

Likewise, although the main embodiments described herein refer to specific values of concentrations of potassium, the claimed method can be applied, in an equivalent manner in the invention, for any other values of said concentrations, with no restriction as to the scope thereof. Therefore, the use of IC₅₀ is to be considered as being extensive to other values of concentration in the scope of interpretation of the invention. In this same sense, it is also possible to modify, with no loss of equivalence, the described method including the use of mutations in I_{Kr} or hERG channels.

In the same way, the preferred embodiments of the method of the invention relate to a selection of drugs to be characterized by means of a simulation of a plurality of compounds. In this sense, said selection of drugs is considered applicable both to known (or real) compounds and to virtual compounds (for example, the formulation of which is generated randomly or pseudorandomly under certain computational rules), with no limitation as to the scope of protection of the invention. Therefore, and in a manner equivalent to that of the mentioned preferred embodiments, in the case of using real compounds, the electrical stimulation voltage clamp protocols would preferably be applied experimentally to obtain the values of IC₅₀ and τ, which will be the input data for the described method. And additionally, it is also possible to obtain the value of τ from the evolution of the tail current.

### DESCRIPTION OF THE DRAWINGS

The above and other features and advantages will be more fully understood from the detailed description of the invention, as well as from the preferred embodiments in reference to the attached drawings, in which:

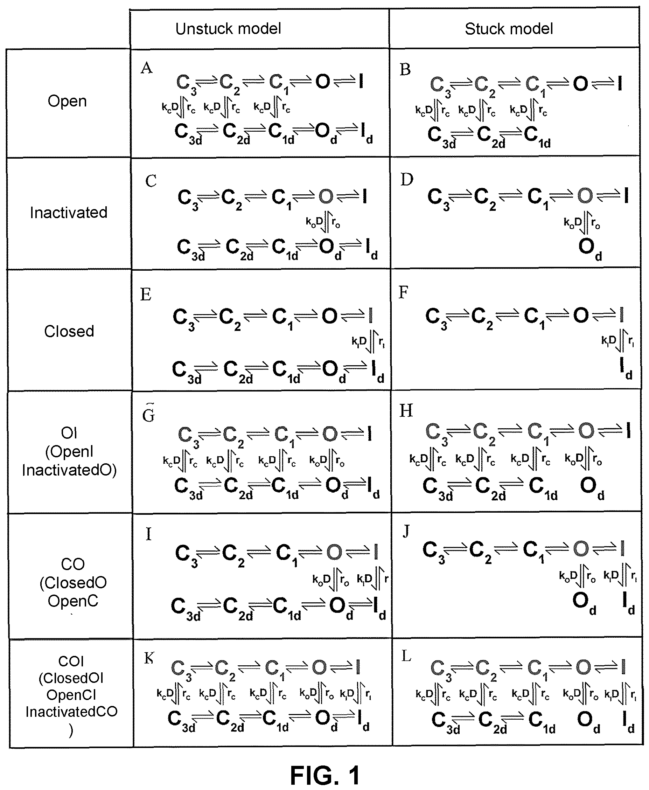

- FIG. 1 shows different I_{Kr} Markov models used in the method of the invention, according to a preferred embodiment thereof. More specifically, the figure represents the drug-bound states (C_{3d}, C_{2d}, C_{1d}, O_{d}, and I_{d}) and the drug-free states (C₃, C₂, C₁, O, and I). k_{c}, kₒ and kᵢ are the diffusion rates for the closed, open, and inactivated states, respectively. R_{c}, rₒ, and rᵢ are the dissociation rates for the closed, open, and inactivated states, respectively. D is the concentration of the drug. The column on the left represents the names of the possible interactions between the drugs and the channels for each model.

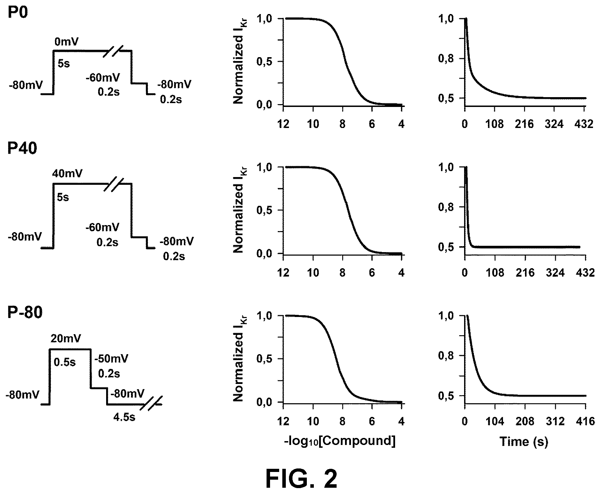

- FIG. 2 shows different plots representing the effects of the three voltage clamp protocols: P0 (top), P40 (center) and P-80 (bottom), depiction of the voltage protocols (left), Hill plots of a given virtual compound with the three protocols (center) and the evolution of the peak tail current (normalized) at the corresponding concentration IC₅₀ (right).

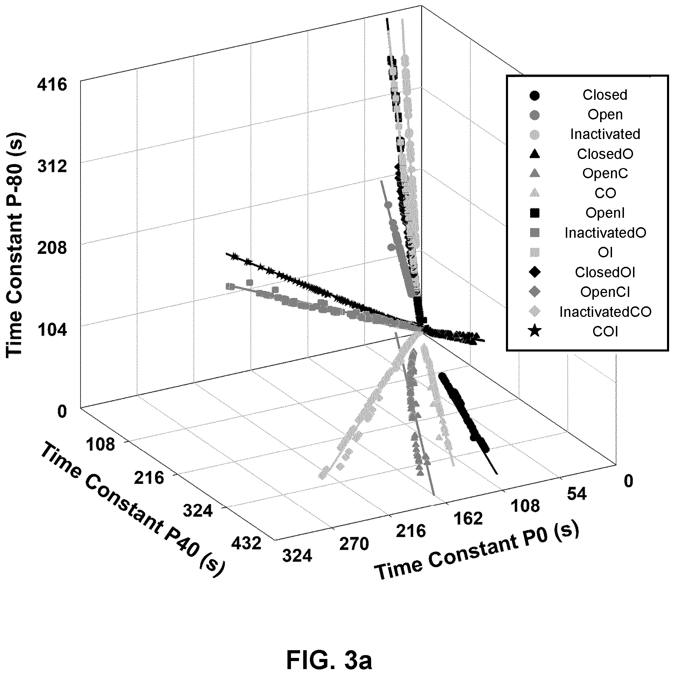

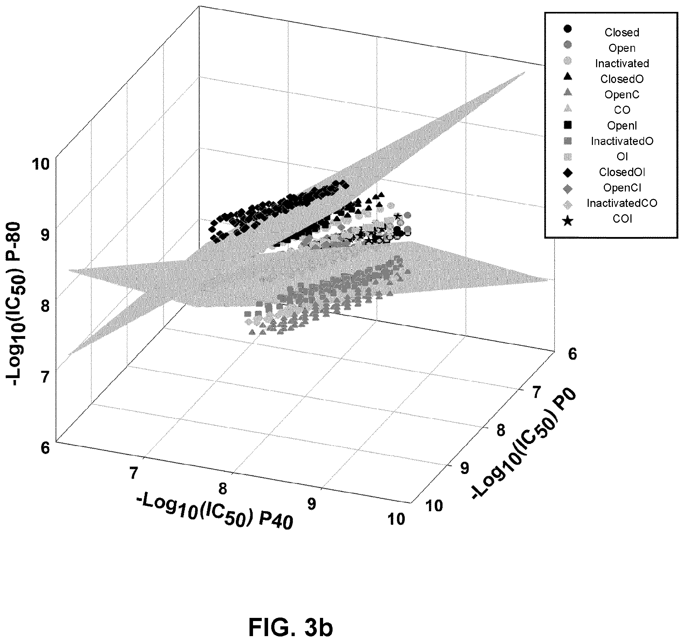

- FIG. 3 corresponds to a flow chart of the different steps of a classifier used in the method of the invention, according to a preferred embodiment thereof.

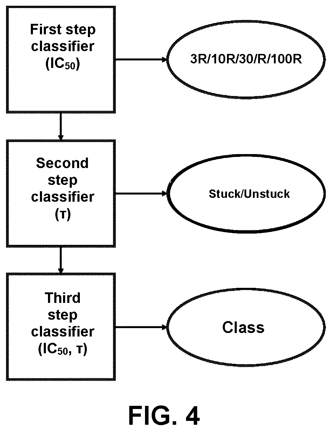

- FIG. 4 shows different examples of three-dimensional depictions of the parameters extracted from the drug simulations by means of the method of the invention, according to a preferred embodiment thereof. It depicts the time constants of a subset of compounds of each class and the corresponding interpolated lines (colors) used in the second and third step of the classifier (FIG. 4a) and values of the IC₅₀ of another subset of compounds together with two decision surfaces (FIG. 4b).

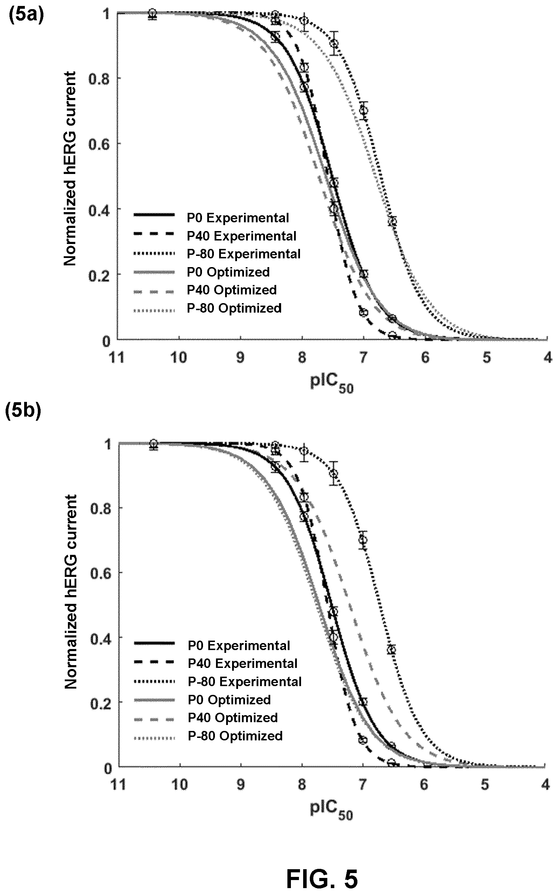

- FIGs. 5a-5b show several Hill plots for dofetilide, obtained with the three voltage protocols, P0 (continuous lines), P40 (discontinuous lines), and P-80 (dotted line), experimentally (black lines) and with two dynamic models (gray lines): the one obtained by means of the present invention (FIG. 5a) and the one proposed by CiPA [Li *et al.* 2017] (FIG. 5b).

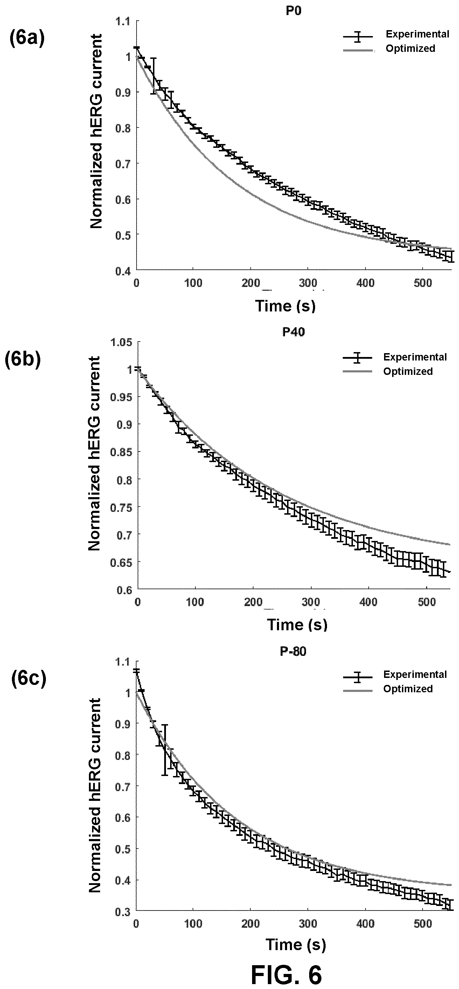

- FIGs. 6a-6c show the evolution of the peak tail current (normalized) at the concentration closest to the IC₅₀ obtained experimentally (black lines) and with the dynamic model obtained by means of the present invention (gray lines) for the three protocols: P0 (FIG. 6a), P40 (FIG. 6b), and P-80 (FIG. 6c).

### DETAILED DESCRIPTION OF THE INVENTION

A detailed description of the invention is set forth below based on the figures of the present document. Said description is provided for purposes of illustrating, but not limiting, the claimed invention.

As described in the preceding sections, a first object of the invention relates to a method for modelling dynamic interactions between compounds and I_{Kr} or hERG channels. Said method advantageously comprises performing the next steps:
- Modelling the I_{Kr} interaction.
- Selecting the compounds.
- Defining stimulation protocols.
- Obtaining results (time constants and values of IC₅₀).
- Classifying.
- Optimizing (optional).

Each of the steps involved in the method will be explained below for a preferred embodiment thereof applied to the interaction of compounds with the I_{Kr} channel (although the interaction with the hERG channel would be equivalent by means of the necessary variations, although said variations are considered accessible to one skilled in the art as they are not themselves a novel object of the invention, but rather the general steps of the method applied to any of said I_{Kr} or hERG channels).

### Modelling the interaction of the drug with I_{Kr}:

In the preferred described embodiment, the I_{Kr} is simulated using an I_{Kr} Markov chain model of a human ventricle, such as, for example, the one proposed in M. Fink et al., "Contributions of HERG K+ Current to Repolarization of the Human Ventricular Action Potential", Prog. Biophys. Mol. Biol. (2008), vol. 96, pp. 357-376, although other models based on Markov chains are likewise implementable without departing from the scope of the invention. In this embodiment, said model comprises five states: three closed states, (C₃, C₂, C₁), an open state (O), and an inactivated state (I). Likewise, to simulate the interaction between the drug and the channel (in this case, the I_{Kr} channel), five new states (C_{3d}, C_{2d}, C_{1d}, O_{d}, and I_{d}) are included, as proposed for example in J. Gomis-Tena et al., "When Does the IC50 Accurately Assess the Blocking Potency of a Drug?", J. Chem. Inf. Model (2020), 60, 3, 1779-1790. All the configurations of the channels considered are shown in FIG. 1.

### Selecting the compounds:

The next step of the method comprises a simulation of the drugs which, in the described embodiment, is based on a set of 26000 virtual drugs, distributed in 104 groups with different kinetics and affinities for the conformational states of the I_{Kr} channel. These groups are generated preferably taking three aspects into account: i) the states to which the drug binds and unbinds and the preferred binding state, which gives rise to thirteen classes, ii) the ratio between the dissociation rate of the preferred and of the non-preferred state, which is arbitrarily set at 3, 10, 30, or 100 as reference threshold values (although other selections are likewise implementable) and, lastly, iii) the ability of bound channels to change their conformational state without becoming separated from the compound, such that an unstuck model is formulated if this ability is exhibited and a stuck model is not exhibited. The thirteen classes are named according to the binding state: Open, Inactivated, and Closed are compounds interacting only in the open, inactivated, and closed states, respectively. OpenI, InactivatedO, and OI are drugs binding to the open and inactivated states, with preference for the open, inactivated state, and with the same preference for both states, respectively. OpenC, ClosedO, and CO represent drugs binding to the closed and open states simultaneously, but with a higher affinity for the open, closed state and with an identical affinity for both states, respectively. Lastly, OpenCl, InactivatedCO, ClosedOl, and COI are compounds binding simultaneously to the three states with a higher affinity for the open, inactivated, or closed states and with the same affinity for all of them, respectively.

Additionally, in a particular embodiment of the invention, it is possible to create additional check groups to verify the operation of the system using another random seed. Thus, in the previously proposed example, another eight different sets of 650 check compounds (variants in which the channels bound to the compound may or may not change their state without becoming separated (unstuck model and stuck model, respectively) of 13 classes and 50 drugs per class) are created.

### Stimulation protocols:

In this step of the method, the voltage clamp protocols used for the simulation are defined. The example described in this embodiment of the invention uses three known voltage clamp protocols published in the paper by Gomis-Tena *et al.* (2020) referenced above, referred to as P40, P0, and P-80, which maximize the likelihood of the channels occupying each conformational state (see FIG. 2, left column). Said protocols essentially consist of a variable voltage conditioning pulse (in the example, 5 s for P0 and P40 and 4.5 s for P-80), followed by a test pulse (in the example, a pulse of 0.2 s at -60 mV, repeated at 5.4 s intervals from a potential of -80 mV). Likewise, the conditioning pulse is performed at a potential of -80, 0, and 40 mV for P-80, P0, and P40, respectively. In the case of P-80, a pre-pulse of 0.5 ms at 20 mV is incorporated, and the test pulse of 0.2 s is set at -50 mV. The value of the temperature (22 °C in the example), the intracellular potassium concentration (130 mM in the example) and extracellular potassium concentration (4 mM) in the example are set within the definition of the protocols. The values of times, potential, temperatures and concentrations may vary in different embodiments of the invention, with no limitation as to the scope thereof.

The corresponding concentration-response plots, also referred to as Hill plots, are obtained from the voltage clamp protocols. In the described example, these plots are created by plotting the steady state normalized peak tail current as a function of the common logarithm of the concentration of the drug (see FIG. 2, middle column), following that described in the publication by Gomis-Tena *et al.* (2020). Additionally, the normalized peak tail current of I_{Kr} at concentration IC₅₀ is analyzed as a function of the number of pulses or on time (FIG. 2, right column).

### Obtaining results:

The three values of the time constant (τ_{P-80}, τ_{P0}, τ_{P40}) and the three values of IC₅₀ (IC_{50,P-80}, IC_{50,P0}, IC_{50,P40}) for all the training compounds are calculated from previously defined simulation parameters, depicted in 3D scatter plots (see FIGs. 3a and 3b, respectively). In the preferred embodiment now described by way of example, it can be seen that the distributions of the value of IC₅₀ and the time constants of all the classes resemble linear trends. These parameters are used to train a classifier which comprises in this embodiment three sub-steps, as depicted in the diagram of FIG. 4. To that end, the IC₅₀ and the values of the time constant are required, and support vector machines (SVMs) and line interpolation are preferably used. The mentioned sub-steps are described in detail below:
- First, the difference in dissociation rates between the preferred state and the other states in which the channel interacts with the compound is obtained. The compounds are classified according to their dissociation rate with respect to the non-preferred binding state (in the present example, 3R, 10R, 30R, and 100R when the dissociation rate of the compound is 3, 10, 30, or 100 times greater for the non-preferred binding state, respectively). For this step, the value of IC₅₀ is used, being interpolated on a line using all the points for each of the 104 possible classifications (3R, 10R, 30R, or 100R, if the channels bound to the compound can change state or not and the 13 final classes, i.e., 4x2x13=104) of the training group. The criterion used for the classification is the distance between the point defined by the three values of IC₅₀ corresponding to the compound and all the interpolated lines, where the compound is assigned to the group corresponding to the closest interpolated line. The ratio of the dissociation rates is therefore determined after this sub-step (see FIG. 4b).
- Secondly, it is ascertained whether the compound allows the drug bound channels to change state or not without becoming separated from the drug. The time constants (τ) are used in this sub-step, and the method is similar to the one used in the first sub-step. In this case, the lines are interpolated for each of the 26 possibilities (whether or not the drug bound channels change their state and the 13 classes, that is, 2x16=26). FIG. 3a shows a three-dimensional depiction of the time constants and the interpolated lines for the drugs 30R, in the variants which allow changing state to the drug bound channels. The distance between the compound and all the lines is calculated, and it is decided that the drug allows the drug bound channels to change their state if the closest line corresponds with compounds that have this characteristic. After this sub-step, the compound is assigned to one of eight groups, which are the result of the four ratios and of the possibility that the compound allows changing state to the channel without becoming separated or not (4x2=8).
- Thirdly, it is assigned the class among the 13 possibilities, according to the above criteria.

In the particular embodiment of the invention, the complete classifier according to the preceding steps correctly classified 88.39% of the compounds, depending on whether they allowed the change of state in the drug bound channels, and ascertained the class in 92.05% of the cases.

### Optimizer:

Once the characteristics of the compounds have been identified, the association and dissociation rates are calculated for each state to which the compound interacts with the channel. To that end, an iterative optimization tool is preferably used, based for example on the Nelder-Mead simplex algorithm. The parameters of said tool are calculated taking into account the characteristics found by the classifier in the previous step. The optimizer adjusts the models of the compounds with the four possibilities: 3R, 10R, 30R, and 100R. Once the optimization of the association and dissociation constants of the four possibilities has been completed, the four errors are compared and the model having the lowest error is selected. In all cases, the solution provided by the optimization tool is the one having the correct ratio of the dissociation rates.

In addition to the method of the invention, another main object thereof relates to a computing system (for example, a computer, although without limitation to other equipment with storage and data processing capacity, such as computing networks or clusters, graphic processing units (GPUs), etc.) comprising software and/or hardware means adapted for implementing a method according to any of the embodiments now described.

Finally, a third object of the invention relates to a computer program comprising instructions configured for being executed in a method according to any of the embodiments described herein, in a corresponding computing system.

### Embodiment of the invention:

As an example of experimental validation of the invention, voltage clamp experiments were performed in HEK cells stably transfected with hERG. The measurements were taken using Nanion SyncroPatch 384i, which is an automated platform in which 384 wells are measured simultaneously. Protocols P-80, P0, and P40 were applied to obtain the values of IC₅₀ and the time constants (τ) for dofetilide, a known drug that blocks I_{Kr} and hERG. The experiments performed with P40, P0, and P-80 took place on the same day, using the same method, the same cells and an identical compound solution. The values of IC₅₀ were thereby obtained for each protocol (IC_{50P-80}, IC_{50P0}, and IC_{50P40}, respectively), and the evolution of hERG block as a function of time at the applied concentration closest to the IC₅₀ for each protocol was recorded by measuring the evolution of the normalized peak tail current as a function of time, the values of the time constants were calculated, and they were considered as τ_{P-80}, τ_{P0} and τ_{P40}, respectively. The three values of IC₅₀ (IC_{50P-80}, IC_{50P0}, and IC_{50P40}) and the three values of the time constant (τ_{P-80}, τ_{P0}, τ_{P40}) were used according to the method described in the present invention to generate the dynamic Markov model for the interaction between dofetilide and the channel. The generated model successfully reproduced experimentally recorded values of IC₅₀ and the evolution of hERG blocking, unlike the dynamic model for dofetilide proposed by CiPA [Li, Z.; Dutta, S.; Sheng, J.; Tran, P. N.; Wu, W.; Chang, K.; Mdluli, T.; Strauss, D. G.; Colatsky, T., "Improving the In Silico Assessment of Proarrhythmia Risk by Combining HERG (Human Ether-à-Go-Go-Related Gene) Channel-Drug Binding Kinetics and Multichannel Pharmacology", Circ.: Arrhythmia Electrophysiol. 2017, 10, No. e004628], as depicted in FIGs. 5a and 5b, respectively. Thus, said FIGs. 5a-5b show the different Hill plots for dofetilide, obtained with the three voltage protocols, P0, P40, and P-80, experimentally (solid lines) and with two dynamic models (discontinuous lines): the one obtained by means of the present invention (FIG. 5a) and the one proposed by CiPA [Li *et al.* 2017] (FIG. 5b).

In turn, FIGs. 6a-6c show the evolution of the peak tail current (normalized) at the concentration closest to the IC₅₀ obtained experimentally (continuous lines) and with the dynamic model obtained by means of the present invention (discontinuous lines) for the three protocols: P0 (FIG. 6a), P40 (FIG. 6b), and P-80 (FIG. 6c).

These results constitute proof of concept of the potential value of the method of the invention for characterizing, modelling, and simulating dynamic interactions between drugs and the I_{Kr} or hERG channels, contributing to improving the pre-clinical evaluation of the proarrhythmic risk of drugs inhibiting I_{Kr} and the efficacy of antiarrhythmic drugs blocking I_{Kr}.

## Claims

1. A computer-implementable method for the characterization of dynamic interactions between drugs and I_{Kr} or hERG channels, wherein said method comprises performing the next steps:
- modelling the interaction between I_{Kr} or hERG and the drugs, wherein said modelling comprises generating a Markov chain model;
- selecting a set of drugs to be **characterized by** means of a simulation of a plurality of compounds distributed in groups with different kinetics and affinities for the conformational states of the I_{Kr} or hERG channels, wherein the distribution of the groups is based on the following criteria:
∘ the states to which the drug binds and unbinds and the preferred binding state;
∘ the ratio between the dissociation rate of the preferred and of the non-preferred state, which is arbitrarily set in a plurality of threshold values;
∘ the ability of bound channels to change their conformational state without becoming separated from the compound;
- defining a plurality of electrical cell stimulation voltage clamp protocols, with their corresponding concentration-response distributions being obtained;
the method being **characterized in that** it further comprises the next steps:
- obtaining values of drug concentration at which the half blocking of an ionic current (IC₅₀) takes place and of the time constants (τ) of the normalized tail current upon application of a value of drug concentration substantially equal to the corresponding IC₅₀ for each voltage clamp protocol and for the plurality of compounds simulated in the step of modelling drugs;
- interpolating the IC₅₀ values with a line based on each of the threshold values, on whether or not the drug bound channels can change state while the compound is bound, and on its preferred binding state;
- interpolating the τ values with a line based on each of the threshold values, on whether or not the drug bound channels can change their state while the compound is bound, and on its preferred binding state;
- classifying the compounds by performing the following sub-steps, performed by means of SVMs and linear interpolation:
o obtaining the difference in dissociation rates between the preferred state and the other states to which each compound is bound, and classifying the compounds according to their dissociation rate with respect to the non-preferred binding state, wherein each compound is assigned to the group corresponding to the closest interpolated line;
∘ assigning each compound to the group corresponding to the closest interpolated line of its time constant (τ), calculating the distance between each compound and all the lines, and determining if the drug allows the drug bound channels to change state if the closest line corresponds to compounds that have this characteristic; and
∘ assigning each compound to a class, characterizing it based on the states to which it binds and unbinds, and on its preferred binding state, according to the results obtained in the previous sub-steps.

2. The method according to the preceding claim, wherein during the step of selecting drugs, the group distribution criterion relating to the ability of drug bound channels to change their conformational state without becoming separated from the compound comprises the formulation of a Markov chain model referred to as a unstuck if said ability is exhibited, or of a Markov chain model referred to as stuck if said ability is not exhibited.

3. The method according to any of the preceding claims, wherein the step of modelling the I_{Kr} or hERG interaction comprises the implementation of a Markov chain model with five drug-free states: three closed states (C₃, C₂, C₁), an open state (O), and an inactivated state (I), and up to five additional drug-containing states (C_{3d}, C_{2d}, C_{1d}, O_{d}, and I_{d}).

4. The method according to the preceding claim, wherein the distribution of groups during the step of modelling drugs is based on the following criteria:
- the states to which the drug binds and unbinds and the preferred binding state;
- the ratio between the dissociation rate of the preferred and of the non-preferred state, which is arbitrarily set in a plurality of threshold values;
- the ability of drug bound channels to change their conformational state without becoming separated from the compound, distinguishing between a stuck model and an unstuck model.

5. The method according to the preceding claim, wherein the distinction of the compounds according to their binding state comprises the following classes: Open, Inactivated, Closed, OpenI, InactivatedO, OI, OpenC, ClosedO, CO, OpenCl, InactivatedCO, ClosedOI and COI.

6. The method according to any of the preceding claims, wherein the step of modelling the I_{Kr} or hERG interaction comprises creating additional check groups using a random seed and generating variants in which the channels bound to the compound may or may not change state without becoming separated.

7. The method according to any of the preceding claims, wherein the step of defining electrical stimulation protocols comprises the use of protocols comprising, for a given value of temperature and a given value of intracellular and extracellular potassium concentration:
- a variable voltage conditioning pulse;
- a test pulse;
- optionally, a pre-pulse.

8. The method according to the preceding claim, wherein the electrical stimulation protocols comprise protocols P40, P0, and P-80.

9. The method according to any of claims 7-8, wherein the step of defining electrical stimulation protocols comprises obtaining concentration-response distributions by means of plotting the steady state normalized peak tail current as a function of the common logarithm of the concentration of the drug, and the normalized peak of the current I_{Kr} at concentration IC₅₀ is analyzed as a function of the number of pulses or of time.

10. The method according to any of the preceding claims, comprising an additional step of optimization wherein, once the compounds are characterized, the association and dissociation constants for each state of the channel where each compound interacts are calculated and processed by means of an iterative optimization algorithm.

11. The method according to the preceding claim, wherein the iterative optimization algorithm is based on the Nelder-Mead simplex algorithm.

12. The method according to any of the preceding claims, comprising the use of mutations in the I_{Kr} or hERG channels.

13. The method according to any of the preceding claims, wherein the plurality of compounds corresponding to the drugs comprises real compounds, and/or virtual compounds the formulation of which is generated randomly or pseudorandomly under one or more computational rules.

14. A computing system comprising software and/or hardware means adapted for implementing a method according to any of the preceding claims.

15. A computer program comprising instructions configured for being executed in a method according to any of claims 1-13 in a system according to claim 14.
